Europäisches Patentamt

European Patent Office

Office européen des brevets

(19)

(11) **EP 1 199 302 B1**

(12) **EUROPÄISCHE PATENTSCHRIFT**

(45) Veröffentlichungstag und Bekanntmachung des Hinweises auf die Patenterteilung:
**01.09.2004 Patentblatt 2004/36**

(51) Int Cl.7: **C07C 253/00**

(21) Anmeldenummer: **01124231.0**

(22) Anmeldetag: **15.10.2001**

(54) **Kontinuierliches Verfahren zur Cyanalkylierung von Verbindungen mit einer oder mehreren NH-Funktionen**

Continuous cyanoalkylation process of compounds having one or more NH functions

Procédé de cyanoalkylation en continu de composés ayant une ou plusieurs fonctions NH

(84) Benannte Vertragsstaaten:
**BE DE NL**

(30) Priorität: **16.10.2000 DE 10051196**

(43) Veröffentlichungstag der Anmeldung:
**24.04.2002 Patentblatt 2002/17**

(73) Patentinhaber: **BASF AKTIENGESELLSCHAFT**
**67056 Ludwigshafen (DE)**

(72) Erfinder:
- **Greindl, Thomas, Dr.**
  **67098 Bad Dürkheim (DE)**
- **Braun, Gerold, Dr.**
  **67071 Ludwigshafen (DE)**
- **Wirsing, Friedrich, Dr.**
  **67346 Speyer (DE)**
- **Krug, Georg**
  **69509 Mörlenbach (DE)**

(74) Vertreter: **Isenbruck, Günter, Dr. et al**
**Isenbruck, Bösl, Hörschler, Wichmann, Huhn,**
**Patentanwälte**
**Theodor-Heuss-Anlage 12**
**68165 Mannheim (DE)**

(56) Entgegenhaltungen:
| EP-A- 0 102 936 | EP-A- 0 237 513 |
| DE-A- 2 025 632 | US-A- 3 463 805 |
| US-A- 3 984 453 | |

- **'Ullmann's Encyclopedia of Industrial Chemistry, 6. Ed.', Bd. 2, WILEY-VCH Seiten 97 - 13**
- **'Ullmann's Encyclopedia of Industrial Chemistry, 6 Ed., vol. 15, p. 16,17,30,32', WILEY-VCH**
- **'Ullmann's Encyclopedia of industrial Chemistry, 6 Ed., vol. 10, p. 211,212,224', WILEY-VCH**

Bemerkungen:
Die Akte enthält technische Angaben, die nach dem Eingang der Anmeldung eingereicht wurden und die nicht in dieser Patentschrift enthalten sind.

EP 1 199 302 B1

## Beschreibung

Die vorliegende Erfindung betrifft ein in zwei Schritten ausgeführtes kontinuierliches Verfahren zur Cyanalkylierung von Verbindungen mit einer oder mehreren NH-Funktionen durch Umsetzung derselben mit Carbonylverbindungen und Blausäure, bei dem in der ersten Stufe drucklos bei einer Temperatur, die unterhalb des Siedepunktes des Reaktionsgemisches liegt, gearbeitet wird.

Es ist bekannt, NH-Funktionen mit Carbonylverbindungen und Blausäure zu

$$\text{>N-H + R-CHO + HCN} \rightarrow \text{>N-CHR-CN + H}_2\text{O}$$

Cyanalkylieren. Die Cyanalkylierungsreaktion verläuft nach folgendem Schema:

Die Reaktion ist stark exotherm und die Reaktionsgeschwindigkeit steigt mit steigender Temperatur.

Bei Verbindungen, die mehrere NH-Funktionen aufweisen, können alle vorhandenen NH-Funktionen cyanalkyliert werden; es ist jedoch prinzipiell auch möglich, durch geeignete Wahl der Reaktionsbedingungen selektiv nur einen Teil der vorhandenen NH-Funktionen umzusetzen. So ist es z.B. möglich, Ammoniak zu Nitrilotriacetonitril N $(CH_2CN)_3$ oder zu Iminodiacetonitril $HN(CH_2CN)_2$ umzusetzen.

Eine Reihe von Verbindungen, die durch Cyanalkylierung hergestellt werden können haben eine große technische Bedeutung erlangt. Sie eröffnen einen Weg zur Herstellung von Aminocarbonsäuren und insbesondere von Stickstoff enthaltenden Polycarbonsäuren, die in großem Umfang als Komplexbildner z.B. bei der Herstellung von Waschmitteln eingesetzt werden.

Es sind daher bereits zahlreiche Vorschläge zur großtechnischen Gewinnung von Cyanalkylaminoverbindungen publiziert worden. Insbesondere hat man sich um eine Steigerung der Ausbeuten bemüht, weil bei den großen, technisch produzierten Mengen bereits relativ geringe Erhöhungen der Ausbeute große wirtschaftliche Vorteile mit sich bringen können. Auch hat man sich um Verfahrensvereinfachung bemüht, insbesondere ist auch bereits versucht worden, die Herstellung von Cyanalkylaminen in kontinuierlicher Weise auszuführen.

Wegen seiner sehr großen technischen Bedeutung sind insbesondere für die Herstellung von Nitrilotriacetonitril (NTN) kontinuierliche Verfahren vorgeschlagen worden.

Aus der US-A-3,907,858 ist ein kontinuierliches Verfahren zur Herstellung von NTN bekannt, bei dem in einem Rohrreaktor bei Temperaturen oberhalb 120°C und Drücken von 135 bis 790 kPa gearbeitet wird. Hierbei treten jedoch erhebliche Korrosionsprobleme auf und auch die Ausbeute ist unbefriedigend, weil unter diesen Bedingungen bereits in erheblichem Umfang eine Verseifung der Cyangruppen eintritt oder auch andere Nebenreaktionen, wie Mehrfach-Cyanmethylierungen und reduktive Methylierungen stattfinden.

Bei einem aus der US-A-3,463,805 bekannten Verfahren wird die Reaktion adiabatisch in einem Rohrreaktor durchgeführt, wobei, ausgehend von einer Starttemperatur zwischen 0 und 130°C, eine Temperaturerhöhung von 50 bis 100°C und eine Druckerhöhung von 100 bis 300 kPa eintritt. In einer Variante dieses Verfahrens wird ein Teil der freiwerdenden Wärme zur Vorwärmung der Ausgangsstoffe verwendet. Dieses Verfahren hat den erheblichen Nachteil, daß es sehr schwierig zu steuern ist und durch einen positiven Rückkopplungseffekt weit aus dem gewünschten Reaktionsbereich ausbrechen kann. Dies hat dann nicht nur eine Verschlechterung der Quantität und der Qualität des erzeugten Produkts zur Folge, sondern kann auch zu Sicherheitsproblemen führen.

Ein dreistufiger kontinuierlicher Prozeß ist aus der US-A-3,925,448 bekannt, wobei in der ersten Stufe Ammoniumsulfat mit Formaldehyd, in der zweiten Stufe das Produkt der ersten Stufe bei 93°C mit Blausäure jeweils in einem Kreislaufreaktor umgesetzt wird und anschließend noch eine Nachreaktion ausgeführt wird. Die Ausbeute liegt bei diesem Verfahren allerdings nur bei ca. 90 % der Theorie.

Aus der EP-B-0 102 936 ist ein weiteres Verfahren zur Herstellung von NTN bekannt. Bei diesem Verfahren wird eine Mischung aus Hexamethylentetramin, Formaldehyd und Blausäure auf einen pH-Wert unter 1 eingestellt und zunächst bei einer Temperatur von 90 bis 120°C in einem Reaktor, vorzugsweise einem Kreislaufreaktor und anschließend bei einer Temperatur von 95 bis 120°C in einem einmal durchströmten, dicht gepackten Röhrenreaktor überführt und zur Reaktion gebracht. Nach Durchlaufen des gepackten Röhrenreaktors wird das Reaktionsgemisch noch durch einen Rücklaufreaktor geführt, bevor es in einen Kristallisator eintritt, wo die Kristallisation des entstandenen NTN erfolgt. Alle Reaktoren sind als doppelwandige Röhren ausgeführt, deren Doppelwandung von einem Wärmeträger durchströmt wird, der für die Temperaturregulierung des Systems erforderlich ist. Dieses Verfahren liefert in der Regel ca. 88 bis 93 % Ausbeute. Die Ausbeute kann, bezogen auf Ammoniak, bis auf etwa 97,5 % gesteigert werden, wenn ein Blausäureüberschuß von 16 bis 17 % eingesetzt wird. Der Einsatz eines so hohen Blausäureüberschusses ist für ein technisches Verfahren ein sehr erheblicher Nachteil. Als weiterer Nachteil dieses Verfahrens sind die erheblichen Investitionskosten für doppelwandige Reaktoren und die Systeme für die Temperaturführung zu nennen.

Aus der DE-A-2 025 632 ist ein zweistufiges, kontinuierliches Verfahren zur Herstellung von Nitrilotriacetonitril bekannt, bei dem in einem ersten Reaktor eine wässrige Lösung eines Ammoniumsalzes in einem stöchiometrischen Überschuss von 5 bis 40 % mit wässrigem Formaldehyd bei 50 bis 110°C 2 Sekunden bis 4 Minuten lang zur Reaktion gebracht wird und das erhaltene Zwischenprodukt in einem zweiten Reaktor mit einer stöchiometrischen Menge Cyanwasserstoff unter einem Druck von 3 bis 25 bar bei einer über dem Schmelzpunkt von Nitrilotriacetonitril liegenden Temperatur umgesetzt wird. Das Verfahren, ist speziell auf die Herstellung von Nitrilotriacetonitril ausgerichtet. Es ist mit einer aufwendigen Aufarbeitung der Mutterlauge, die neben Nitrilotriacetonitril noch Glycolsäurenitril, Methylaminodiacetonitril und Methylen-bis-aminodiacetonitril enthält, gekoppelt. Die Ausbeute an Nitrilotriacetonitril beträgt maximal 93,4 % der Theorie.

Aus der EP-A 0 237 513 ist ein Verfahren zur Herstellung von Iminodiacetonitril bekannt, bei dem wässrige Lösungen von Formaldehyd und von Ammoniak oder eines Ammoniumsalzes einer nicht oxidierenden Säure und Blausäure gleichzeitig einem Reaktor in den Molverhältnissen Formaldehyd zu Blausäure von 0,8 bis 1,2 und Ammoniak zu Blausäure von 0,4 bis 0,6 zugeführt und bei Temperaturen zwischen 30 und 65°C und einem pH-Wert von 1,5 bis 5,5 mit einander umgesetzt werden. Nachteile dieses Verfahrens sind, dass es sich nicht für die Herstellung anderer Cyanalkylierungsprodukte als Iminodiacetonitril eignet, dass die Ausbeute an Iminodiacetonitril mit maximal 74,2 % der Theorie für die Herstellung im industriellen Maßstab nicht ausreicht und dass verschiedene Nebenprodukte, wie Methylen-bis-iminodiacetonitril, Aminoacetonitril und Glycolsäurenitril anfallen und zu entsorgen sind.

Aus der US-A 3 984 453 ist ein kontinuierliches, mehrstufiges Verfahren zur Herstellung von Methylen-bis-iminodiacetonitril, Nitrilotriacetonitril und Nitrilotriessigsäure bekannt. Bei diesem Verfahren wird in einer ersten Verfahrensstufe ein Addukt aus Ammoniak und Formaldehyd hergestellt, die erhaltene wässrige Phase stark angesäuert und das Addukt bei Temperaturen unterhalb 50°C mit Blausäure zu Methylen-bis-iminodiacetonitril umgesetzt. Dieses Produkt kann aus dem Prozess ausgeschleust oder in einer dritten Verfahrensstufe bei einer Temperatur von 50-85°C mit den erforderlichen Mengen von Formaldehyd und Blausäure zu Nitrilotriacetonitril umgesetzt werden. Bei den einzelnen Verfahrensstufen werden vorzugsweise stöchiometrische Mengen der Reaktanden eingesetzt. Das bei diesem Verfahren erhaltene Methylen-bis-iminodiacetonitril kann auch mit Alkalihydroxid und Alkalicyanid direkt zu einem Alkalisalz der Nitrilotriessigsäure umgesetzt werden. Das Verfahren liefert sehr gute Ausbeuten von bis zu 97-98 % d. Th. an Nitrilotriacetonitril, bis zu 96 % d. Th. Methylen-bis-iminodiacetonitril und bis zu 71 % d. Th. Alkali - nitrilotriacetat. Es eignet sich allerdings nicht für die Herstellung partiell cyanalkylierter Amine.

Es wurde nun überraschend gefunden, dass es möglich ist, Cyanalkylierungen mit vergleichsweise geringem technischen Aufwand und in sehr guten Ausbeuten durchzuführen, wenn man den Hauptteil der Reaktion bei relativ niedriger Temperatur in einem drucklosen System ausführt und nur der Restumsatz in einem einfachen Röhrenreaktor ohne aktive Zu- oder Abfuhr von Wärme durchführt.

Ein Gegenstand der vorliegenden Erfindung ist somit ein Verfahren zur Cyanoalkylierung von Ammoniak oder organischen Verbindungen mit einer oder mehreren NH-Funktionen durch Umsetzung besagter Verbindungen mit einer Carbonylverbindung und Blausäure in einem geeigneten Lösungsmittel, das dadurch gekennzeichnet ist, daß eine NH-Funktionen enthaltende Verbindung der Formel I

$$R_a\text{-}NH_{(3-a)} \qquad\qquad (I),$$

worin R ein gegebenenfalls substituierter linearer, verzweigter oder cyclischer Alkylrest mit 1 bis 8 C-Atomen oder Aryl mit 6 bis 10 C-Atomen ist, a eine der Ziffern 0, 1 oder 2 bedeutet und die Verbindung der Formel I insgesamt 1 bis 10 Kohlenstoffatome hat, mit einem Aldehyd der Formel II

$$R^2\text{-}CHO \qquad\qquad (II),$$

worin $R^2$ Wasserstoff oder ein gegebenenfalls substituierter linearer oder verzweigter Alkylrest mit 1 bis 6 C-Atomen ist im Molverhältnis Verbindung der Formel I zu Verbindung der Formel II von 0,2 bis 2,0 und Blausäure umsetzt, wobei die Reaktion zunächst in einem oder mehreren kaskadierten Reaktoren drucklos bei einer Temperatur $T_1$ von unter 85°C bis zu einem Umsetzungsgrad von mindestens 60 %, bezogen auf Blausäure, und anschließend in einem oder mehreren kaskadierten unter Druck arbeitenden Reaktoren bei einer Temperatur $T_2$, die unterhalb 150°C liegt, bis zu einem Umsetzungsgrad von mindestens 90 %, bezogen auf Blausäure, ausgeführt wird.

NH-Funktionen im Sinne dieser Erfindung sind alle direkten Bindungen eines Wasserstoffatoms an ein Stickstoffatom. Ein Stickstoffatom kann bis zu drei NH-Funktionen aufweisen ($NH_3$); eine $-NH_2$-Gruppe weist zwei, eine =NH-Gruppe eine NH-Funktion auf. Natürlich können die Stickstoffatome von Verbindungen, die mehrere Stickstoffatome enthalten, unterschiedliche Anzahlen von NH-Funktionen aufweisen, wobei die Anzahl der an den einzelnen N-Atomen vorhandenen NH-Funktionen null, eins oder zwei betragen kann. Verbindungen mit mehreren NH-Funktionen können diese am gleichen oder an verschiedenen, gegebenenfalls im Molekül vorhandenen N-Atomen enthalten.

Als NH-Funktionen enthaltende Reaktionskomponente wird beim erfindungsgemäßen Verfahren eine Verbindung der Formel I

$$R_a\text{-}NH_{(3-a)} \qquad (I)$$

eingesetzt, worin R ein gegebenenfalls substituierter linearer, verzweigter oder cyclischer Alkylrest mit 1 bis 8 C-Atomen oder Aryl mit 6 bis 10 C-Atomen ist, a eine der Ziffern 0, 1 oder 2 bedeutet und die Verbindung der Formel I insgesamt 1 bis 8 Kohlenstoffatome hat.

Vorzugsweise bedeutet R in Formel I lineares Alkyl mit 1 bis 4, insbesondere 1 oder 2 C-Atomen oder Phenyl. Der Index a steht, sofern R Alkyl ist, vorzugsweise für 1 oder 2, sofern R ein Arylrest ist, für die die Ziffer 1.

Enthält die Verbindung der Formel I 2 Reste R, so können diese im Rahmen der gegebenen Definition gleich oder verschieden sein. Sie können sich in Bezug auf ihre Kettenlänge, ihre Struktur (linear, verzweigt oder cyclisch, aliphatisch oder aromatisch) und/oder ihre Substituenten unterscheiden.

Ferner können zwei Reste R auch miteinander verbunden sein, so daß die NH-Funktion Teil eines Ringgebildes wird.Die Verbindung der Reste R kann entweder durch eine direkte Bindung oder durch ein zweibindiges Brückenglied erfolgen. Geeignete und bevorzugte zweibindige Brückenglieder sind beispielsweise $-CH_2-$, $-NR^3-$ ($R^3$=Wasserstoff oder Alkyl mit 1 bis 4 C-Atomen) oder -O-. Die so entstehenden Ringe weisen vorzugsweise 5 bis 7 Glieder auf.

Als Substituenten der für R stehenden Alkylgruppen kommen die Carboxylgruppe oder deren Salze, die Cyangruppe, eine Aminogruppe $-NH-R^1$, worin $R^1$ Wasserstoff oder Alkyl mit 1 bis 4, vorzugsweise 1 oder 2 C-Atomen ist, Alkoxy mit 1 bis 4 C-Atomen oder Alkylmercapto mit 1 bis 4 C-Atomen in Betracht. Bevorzugt als Substituenten für R sind die Carboxyl- oder Carboxylatgruppe und die Cyangruppe.

Beispiele für Verbindungen der Formel I, die sich sehr gut zur Umsetzung nach dem erfindungsgemäßen Verfahren eignen und zu großtechnisch besonders wichtigen Produkten führen, sind Ammoniak ($NH_3$), Monomethylamin ($CH_3NH_2$), Dimethylamin (($CH_3)_2NH$), Mono- und Diethylamin ($C_2H_5NH_2$, ($C_2H_5)_2NH$), Aminoacetonitril ($NC\text{-}CH_2\text{-}NH_2$), Iminodiacetonitril ($HN(CH_2\text{-}CN)_2$), Aminosäuren wie beispielsweise Glycin ($H_2N\text{-}CH_2\text{-}COOH$), N-Alkylglycin, Alanin ($CH_3\text{-}CH(NH_2)\text{-}COOH$) oder N-Alkylalanin, N-Cyanmethylalanin ($NC\text{-}CH_2\text{-}NH\text{-}CH(CH_3)\text{-}COOH$), Hexamethylenimin, Piperidin, Piperazin, Pyrrolidin, Morpholin.

1,2-Diaminoethan ($H_2N\text{-}CH_2CH_2\text{-}NH_2$) und seine N-Mono-, N-Di- oder N-Trisubstitutionsprodukte können ebenfalls eingesetzt werden, wenn man auf die Temperaturempfindlichkeit der entsprechenden Nitrile Rücksicht nimmt.

Selbstverständlich können auch mehrere verschiedene Verbindungen der Formel I im Gemisch oder nacheinander umgesetzt werden. Auch können entsprechende ungereinigte Rohprodukte oder Mutterlaugen dieser Verbindungen eingesetzt werden. Dies gilt insbesondere für Mutterlaugen, die nach der Abtrennung des Endprodukts anfallen und zum Zweck der Ausbeutesteigerung ganz oder teilweise erneut in die Synthese eingesetzt werden.

Als Carbonylverbindung wird beim erfindungsgemäßen Verfahren ein Aldehyd der Formel II

$$R^2\text{-}CHO \qquad (II)$$

eingesetzt, worin $R^2$ Wasserstoff oder ein gegebenenfalls substituierter linearer oder verzweigter Alkylrest mit 1 bis 6, vorzugsweise 1 bis 3, insbesondere 1 oder 2, C-Atomen ist.

Vorzugsweise wird als Carbonylverbindung Formaldehyd, Acetaldehyd oder Propionaldehyd eingesetzt.

Die beiden Reaktionsabschnitte werden in hintereinandergeschalteten Reaktoren ausgeführt.

Der erste Reaktionsabschnitt wird in einem oder mehreren kaskadierten, drucklos arbeitenden Reaktoren ausgeführt. Die drucklos arbeitenden Reaktoren sind zweckmäßigerweise mit Mischvorrichtungen (z.B. Rührern und ggf. Strömungsbrechern) und Einrichtungen zur Temperatur- und pH-Regulierung versehene Kesselreaktoren.

Insbesondere sind Mischvorrichtungen, wie z.B. schnelllaufende Scheiben- oder Propellerrührer, vorhanden, die es erlauben, anfallende Kristallisate möglichst homogen zu verteilen.

Vorzugsweise wird im ersten Reaktionsabschnitt die Umsetzung bis zu einem Umsetzungsgrad von 60 bis 85 %, vorzugsweise von 70 bis 80 % getrieben, wofür unter den oben beschriebenen Reaktionsbedingungen eine Verweilzeit von 10 bis 400 Minuten, vorzugsweise 10 bis 250 Minuten, insbesondere 20 bis 200 Minuten erforderlich ist.

Der zweite Reaktionsabschnitt wird in einem oder mehreren kaskadierten, unter Druck arbeitenden Reaktoren ausgeführt. Im zweiten Reaktionsabschnitt wird die Umsetzung bis zu einem Umsetzungsgrad von 90 bis 100 %, vorzugsweise von 95 bis 100 % getrieben. Zur Erreichung dieses gewünschten Umsetzungsgrads wird für den zweiten Reaktionsabschnitt eine Verweilzeit von 1 bis 60 Minuten, vorzugsweise 2 bis 20 Minuten, insbesondere 5 bis 15 Minuten eingestellt. Auch die erforderliche Verweilzeit hängt von der Struktur der eingesetzten Verbindungen und des Zielprodukts ab und kann durch einige Vorversuche im Rahmen der angegebenen Grenzen optimiert werden.

Vorzugsweise werden für den zweiten Reaktionsabschnitt Rohrreaktoren eingesetzt, die zweckmäßigerweise mit Einbauten zur Verminderung der Rückmischung versehen sind. Insbesondere werden sie so konstruiert, daß erforderlichenfalls auch der Durchfluß von Emulsionen und Suspensionen möglich ist, d. h. daß die Einbauten neben der Verminderung der Rückmischung auch die Umsetzung inhomogener Reaktionsgemische erlauben.

Durch diese Maßnahme können besonders hohe Umsatzraten erzielt werden. Sie ist deshalb besonders vorteilhaft für die Herstellung wertvoller großtechnischer Produkte mit relativ geringer Löslichkeit, wie z.B. von Nitrilotriacetonitril.

Als besonders vorteilhafte Einbauten haben sich Lochböden erwiesen. Sind im zweiten Reaktionsschritt inhomogene Reaktionsgemische weiter umzusetzen, so wird zweckmäßigerweise die Strömungsgeschwindigkeit so reguliert, daß sich im Bereich der Löcher der Lochböden Scherkräfte einstellen, die ein Absetzen von Inhomogenitäten verhindern bzw. die eine Auflösung vorhandener Ablagerungen bewirken. Gleichzeitig soll die Konstruktion der Lochböden einen unnötig hohen Anstieg der Druckverluste in technisch ungünstige Bereiche vermeiden.

Vorzugsweise sollte die Anordnung und Dimensionierung der Bohrungen in den Lochböden so erfolgen, daß die Ausbildung einer Kernströmung unterbunden wird und über den gesamten Querschnitt des Reaktors eine weitgehend gleichmäßige Strömungsgeschwindigkeit erzeugt wird.

Die Querschnittsverringerung in den Lochböden liegen vorzugsweise im Bereich von 10 bis 95%, insbesondere von 20 bis 80%, die Lochdurchmesser bewegen sich vorzugsweise im Bereich von 0,1 bis 10 mm, insbesondere von 1 bis 5 mm.

Alternativ zu den Lochböden können auch Packungen eingesetzt werden, die eine nur geringe Verringerung des Gesamtquerschnittes verursachen und genügend große Hohlräume zurücklassen, wie z.B. Kugel oder Splitpackungen oder Packungen aus Sieb-oder Raschigringen.

Bei dem erfindungsgemäßen Verfahren ist es auch möglich, mit zeitlich unterschiedlichen Strömungsgeschwindigkeiten zu arbeiten, die ggf. nach einem empirischen Programm gesteuert werden.

Die Wahl der Temperatur $T_1$, die in der Regel zwischen 10°C und dem Siedepunkt des reagierenden Gemisches, vorzugsweise zwischen Raumtemperatur (20 - 25°C) und einer Temperatur liegt, bei der noch keine nennenswerten Blausäureverluste durch Abdampfen auftreten, richtet sich nach der konkreten Aufgabenstellung. In der Regel liegt T, unter 85°C. Bevorzugt sind Temperaturen von 30 bis 60°C.

Soll bei einer Ausgangsverbindung mit mehreren NH-Gruppen nur ein bestimmter Teil der NH-Funktionen cyanalkyliert werden, so wird zweckmäßigerweise eine möglichst niedrige Temperatur $T_1$ gewählt, wodurch die Selektivität der Cyanalkylierung steigt. Es wird somit die Bildung höher cyanalkylierter Verbindungen sowie die Weiterreaktion von bereits entstandenem Produkt, z.B. unter Verseifung zu Amidstrukturen, möglichst gering gehalten. Andererseits führt eine niedrige Temperatur natürlich zu einer Herabsetzung der Reaktionsgeschwindigkeit, was durch eine Verlängerung der Reaktionszeit, d.h. durch Verlängerung der Verweilzeit des Reaktionsgemisches in den Reaktoren auszugleichen ist. Die Temperatur $T_2$ liegt in der Regel zwischen $T_1$ und 150°C. Für die Auswahl der Temperatur im konkreten Einzelfall gelten im Wesentlichen die gleichen Grundsätze wie für die Wahl der Temperatur $T_1$. Ein Vorteil ergibt sich durch eine relativ niedrige Temperatur $T_2$ in der Regel bei der Herstellung von Produkten, bei denen nur ein Teil der vorhandenen NH-Funktionen cyanalkyliert werden soll, bei der Herstellung von temperaturempfindlichen Produkten und in Systemen mit dominanter thermodynamischer Reaktionskontrolle.

Im Prinzip kann $T_2$ auch unter $T_1$ liegen. Voraussetzung hierfür ist, daß die Löslichkeit oder die Dispergierbarkeit aller Komponenten des Reaktionsgemisches es erlauben, daß das Reaktionsgemisch störungsfrei durch die Einbauten des oder der Rohrreaktoren strömen kann. Für den Fall, daß ein Absenkung der Temperatur $T_2$ gegenüber $T_1$ erforderlich oder günstig ist, wird zweckmäßigerweise die Länge des Rohrreaktors vergrößert, um den erwünschten hohen Umsetzungsgrad zu erreichen.

Ein besonderer Vorteil des erfindungsgemäßen Verfahrens, der eine besonders einfache Konstruktion der Reaktoren ermöglicht, besteht darin, daß der zweite Reaktionsabschnitt ohne aktive Zu- und Abfuhr von Wärme ausgeführt werden kann. Sofern im Rohrreaktor bei einer oberhalb $T_1$ liegenden Temperatur gearbeitet werden soll, kann die Selbsterwärmung des Reaktionsgemisches für die gewünschte Temperaturerhöhung ausgenutzt werden. Es ist dann zweckmäßig, nötigenfalls die Wärmeabfuhr durch bekannte, geeignete Wärmedämmungsmaßnahmen herabzusetzen. Alternativ kann der Produktstrom selbstverständlich auch vor dem Eintritt in den zweiten Reaktionsabschnitt auf die gegebenenfalls gewünschte erhöhte Reaktionstemperatur erwärmt werden.

Innerhalb der oben genannten Grenzen der Reaktionstemperaturen wird zweckmäßigerweise der für die aktuelle Reaktion am besten geeignete Temperaturbereich in an sich bekannter Weise durch einige Vorversuche ermittelt.

So ist es beispielsweise vorteilhaft, speziell für die Umsetzung von Formaldehyd mit Verbindungen der Formel I, in der a = 2 ist und beide Reste -R für -CHR$^2$-CN, insbesondere für den Rest -CH$_2$-CN stehen, den Produktstrom vor dem Eintritt in den zweiten Reaktionsabschnitt zweckmäßigerweise auf eine Temperatur über 85°C, vorzugsweise auf 95 bis 135°C, insbesondere auf 110 bis 120°C zu erwärmen.

Sofern mindesten einer der beiden Reste -R oder beide für andere Reste als -CH$_2$-CN stehen, wird der Produktstrom vor dem Eintritt in den zweiten Reaktionsabschnitt zweckmäßigerweise auf eine Temperatur über 40°C, vorzugsweise auf 45 bis 135°C, insbesondere auf 45 bis 95°C erwärmt. Bei Einsatz von Alkanalen liegen die Temperaturen im zweiten Reaktionsabschnitt vorzugsweise unter 100°C.

Das erfindungsgemäße Verfahren kann in vielfacher Weise variiert und an die Bedürfnisse des Betriebs und/ oder die Eigenschaften der Ausgangs- und Endprodukte angepaßt werden.

Die Carbonylverbindung kann als solche oder auch in Form ihres Bisulfitaddukts in die Reaktion eingeführt werden. Vorzugsweise wird die freie Carbonylverbindung eingesetzt. Es ist auch möglich, die Carbonylverbindung und die Blausäure in Form des Cyanhydrins der Carbonylverbindung in die Reaktion einzuführen.

Ferner können die Carbonylverbindung und die NH-Funktionen aufweisende Verbindung in Form ihrer reversibel spaltbaren Umsetzungsprodukte in die Reaktion eingeführt werden. Die NH-Funktionen aufweisende Verbindung kann gewünschtenfalls ganz oder teilweise in Form ihres Salzes eingesetzt werden.

An der Reaktion können mehrere Individuen NH-Funktionen aufweisender Verbindungen beteiligt sein, die entweder als Ausgangsmaterialien in die Reaktion eingeführt werden oder sich im Verlauf der Reaktion bilden durch sukzessive Cyanalkylierung mehrerer vorhandener NH-Funktionen, die an einem oder gegebenenfalls an mehreren verschiedenen, im Molekül enthaltenen N-Atomen vorhanden sein können.

Die verschiedenen Individuen können als Gemisch oder nacheinander in geplanter Abfolge in die Reaktion eingeführt werden.

Selbstverständlich ist auch die Einführung verschiedener Carbonylverbindungen in die Reaktion möglich, sofern die Herstellung von Gemischen der Zielprodukte gewünscht wird. In der Regel werden jedoch technisch einheitliche Ausgangsmaterialien für die Ausführung des erfindungsgemäßen Verfahrens benutzt.

Als Lösungsmittel können für das erfindungsgemäße Verfahren Wasser, niedere Alkanole oder Mischungen davon eingesetzt werden. Bevorzugtes Lösungsmittel ist Wasser.

Um eine möglichst hohe Ausbeute des Zielprodukts zu erreichen, wird zweckmäßigerweise darauf geachtet, daß auch im zweiten Reaktionsschritt noch eine ausreichende Blausäurekonzentration im Reaktionsgemisch vorliegt. Dies verschlechtert nicht die Blausäurebilanz des erfindungsgemäßen Verfahrens weil nicht umgesetzte Blausäure aus der Mutterlauge abdestilliert und in den ersten Reaktionsabschnitt zurückgeführt werden kann.

Zur Durchführung des erfindungsgemäßen Verfahrens wird das Molverhältnis von der die NH-Funktionen enthaltende Verbindung zur eingesetzten Carbonylverbindung in der Regel im Bereich von 0,2 bis 2,0, vorzugsweise von 0,3 bis 1,7 insbesondere von 0,3 bis 1,5 eingestellt.

Das zweckmäßigerweise einzustellende Molverhältnis ist allerdings stark davon abhängig, wieviel der vorhandenen NH-Funktionen substituiert werden sollen. So werden z.B. für die Synthese von NTN (Nitrilo-triacetonitril) pro mol $NH_3$ rechnerisch 3 mol Carbonyverbindung, für Glycinnitril dagegen pro mol $NH_3$ nur etwa 1 mol Carbonylverbindung benötigt. Es ist daher zweckmäßig, das Molverhältnis auf den molaren Anteil der zu substituierenden NH-Funktionen zu beziehen.

Obwohl bei dem erfindungsgemäßen Verfahren die NH-Funktion(en), die Carbonylverbindung und die Blausäure sich im Molverhältnis 1:1:1 miteinander verbinden, kann es in vielen Fällen vorteilhaft sein, dieses Molverhältnis nicht von Anfang an im Reaktionsansatz einzustellen. Vielmehr ist es dann zu bevorzugen, je nach dem herzustellenden Produkt und den eingesetzten Ausgangsstoffen die NH-Funktionen enthaltende Verbindung und die Carbonylverbindung in einem solchen Mengenverhältnis zu mischen, daß pro Mol umzusetzender NH-Funktion 0,5 bis 1,25, vorzugsweise von 0,6 bis 1,20, insbesondere von 0,7 bis 1,1 Mol Carbonylverbindung im Reaktionsgemisch vorliegen.

Innerhalb der angegebenen Mischungsgrenzen richten sich, wie gesagt, die Mischungsverhältnisse nach dem aktuellen Problem. Soll bei einer Verbindung mit mehreren NH-Funktionen nur eine oder ein Teil derselben cyanalkyliert werden, so kann die Selektivität erhöht, d.h. die Bildung eines einheitlichen Reaktionsprodukts erheblich erhöht werden, wenn man an den unteren Grenzen der angegebenen Bereiche arbeitet. Soll eine möglichst vollständige Cyanalkylierung aller in der Verbindung enthaltenen NH-Funktionen erfolgen, so wird zweckmäßigerweise in der Nähe der Obergrenzen der Bereiche gearbeitet.

Es ist auch möglich und gegebenenfalls vorteilhaft, die Dosierung eines oder mehrerer der Reaktionspartner über die Reaktionskaskade verteilt vorzunehmen. Beispielsweise kann in den ersten Reaktor nur 50 % der erforderlichen Menge einer Reaktionskomponente, z.B. der Blausäure, in den zweiten Reaktor die restlichen 50 % zugegeben werden.

Das Molverhältnis von Carbonylverbindung zu Blausäure wird im Reaktionssystem zweckmäßigerweise im Bereich von 0,5 bis 1,2, vorzugsweise von 0,8 bis 1,1, insbesondere von 0,95 bis 1,05 gehalten.

Eine weitere Modifizierung des erfindungsgemäßen Verfahrens besteht darin, daß die NH-Funktionen enthaltende Verbindung zunächst mit der Carbonylverbindung und anschließend mit Blausäure umgesetzt wird. Diese Reaktionsabfolge erweist sich insbesondere in solchen Fällen als günstig, wo die NH-Funktionen aufweisende Verbindungen mit niedrigem Siedepunkt, wie z.B. Ammoniak, eingesetzt werden sollen. So kann beispielsweise die Cyanmethylierung von $NH_3$ besonders günstig durch Umsetzung von Blausäure mit Urotropin erfolgen. Iminodiacetonitril und Nitrilotriacetonitril lassen sich daher auf diese Weise mit besonderem Vorteil herstellen.

Noch eine weitere Modifizierung des erfindungsgemäßen Verfahrens besteht darin, daß die NH-Funktionen enthaltende Verbindung mit einem Addukt der Carbonylverbindung und Blausäure umgesetzt wird. Diese Umsetzung verläuft in vielen Fällen in merklichen Umfang auch parallel zu anderen Reaktionswegen der Cyanalkylierung und kann zur Erhöhung der Ausbeute beitragen, wenn das Addukt, das sich in der Regel als Nebenprodukt neben dem Cyanalkylierungsprodukt bildet, in ausreichendem Maße zurückspaltet. Es ist daher zweckmäßig, die Reaktionsbedingungen, insbesondere im zweiten Reaktionsabschnitt, so zu wählen, daß die Rückspaltung des Addukts begünstigt wird. Isbesondere bei der Herstellung von NTN kann durch eine Reaktivierung des Formaldehyd-cyanhydrins noch eine merkliche Ausbeutesteigerung erreicht werden.

In der Regel werden die beiden Reaktionswege, nämlich

1. die Reaktion einer Additionsverbindung aus der NH-Funktionen aufweisenden Verbindung und der Carbonylverbindung mit Blausäure und
2. die Reaktion einer NH-Funktionen aufweisenden Verbindung mit einem Addukt der Carbonylverbindung und Blausäureals Parallel- oder Nebenreaktionen bei der Durchführung des erfindungsgemäßen Verfahrens simultan verlaufen.

Es ist im Rahmen der Erfindung auch möglich, Amin, Carbonylverbindung und HCN gleichzeitig zu vereinigen. Dies ist insbesondere dann von Vorteil, wenn nur eine Teilsubstitution der vorhandenen NH-Funktionen gewünscht ist. Es kann sogar von Vorteil sein, zunächst Amin und HCN vorzumischen und dann die Carbonylverbindung zuzugeben.

Die Blausäure wird vorzugsweise in freier Form, kann aber auch in Form eines Metallsalzes, vorzugsweise eines Alkalisalzes eingesetzt werden.

Ammoniak und Formaldehyd können in Form von Hexamethylentetramin (Urotropin), Ammoniak und Acetaldehyd in Form von Aldehydammoniak in die Reaktion eingeführt werden.

Die wesentlichen Vorteile des erfindungsgemäßen Verfahrens gegenüber anderen bekannten kontiunierlichen Herstellungsverfahren für NTN, insbesondere auch gegenüber dem aus der EP-B-0 102 936 bekannten Verfahren liegen in der mit einfachen technischen Mitteln und geringen Blausäureüberschüssen zu erzielenden Ausbeutesteigerung. Durch die Verknüpfung von drucklosen Kesselreaktoren mit einem einfachen Rohrreaktor kann der Hauptteil der Reaktion bei milden Temperaturen und unter pH-Haltung durchgeführt werden. Dies hat den Vorteil, daß unerwünschte Nebenreaktionen, insbesondere die Verseifung von bereits gebildetem Produkt, weitgehend vermieden werden kann und daß man eine höhere Selektivität der Reaktion erhält, wodurch die Flexibilität des Verfahrens ganz erheblich gesteigert werden kann, sodaß es der Herstellung sehr verschiedener Cyanalkylierungsprodukte angepaßt werden kann.

Vorteilhaft bei der zweistufigenReaktionsführung ist auch die Möglichkeit der Anwendung eines stationären Blausäureüberschusses. Hieraus resultiert bereits in der ersten Reaktionsstufe ein hoher Umsatzgrad, so daß vor Eintritt des Reaktionsgemisches in den Rohrreaktor der Gehalt an freier Blausäure (Leichtsieder und daher Druckverursacher) in der Regel nur bei ca. 1,5 % liegt. Daher sind die Drücke bei z.B. 115°C mit 150 HPa gegenüber den beim nächstvergleichbaren Verfahren (EP-B- 0 102 936 ) auftretenden Drücken von 500 bis 600 HPa vergleichsweise gering. Ferner ist durch den geringen Anteil an Restreaktion im Rohrreaktor keine aufwendige Temperaturkontrolle erforderlich; die Temperatur steigt in der Regel um nicht mehr als 5°C an. Dadurch wiederum ist eine sehr exakte Einstellung der Reaktionsbedingungen (Temperatur und Druck) möglich. Hieraus ergeben sich niedrigere Investitionskosten und last not least eine höhere Verfahrens- und Anlagensicherheit, da Blausäure in möglichst geringer Menge, bei möglichst geringen Drücken und möglichst geringer verbleibender Wärmeentwicklung gehandhabt wird.

Die erfindungsgemäß hergestellten α-Cyanalkylamine werden überwiegend zu den entsprechenden Carbonsäuren verseift, die auf vielen Gebieten der Technik, vorzugsweise als Komplexbildner z.B. bei der Herstellung von Waschmitteln, zum Einsatz kommen.

Einige Beispiele von technisch sehr bedeutenden, nach dem erfindungsgemäßen Verfahren herstellbaren Verbindungen und deren Folgeprodukte sind auf der nächsten Seite in einer Übersicht zusammengestellt.

Die erfindungsgemäß hergestellten α-Cyanalkylamine sind Ausgangsmaterialien in weiterführenden Syntheseverfahren, insbesondere solchen, deren erster Syntheseschritt in einer Verseifung der Cyangruppen zu Carbonamiden oder Carbonsäuren besteht.

| Primäres Ausgangs material | Cyanalkylierungs-Produkt | Technisch wichtiges Folgeprodukt |
|---|---|---|

$NH_3$

$NH_3$

$NH_3$

$H_3C-NH_2$

Die folgenden Ausführungsbeispiele veranschaulichen die vorliegende Erfindung.

Die für die Durchführung der folgenden Beispiele eingesetzte Labor-Versuchsanlage, besteht aus zwei kaskadierten 2-1-Vierhals-Glaskolben mit Rührern, Thermometer, pH-Elektroden, paraffinverschlossenen Gärröhrchen, welche über bei 0°C betriebene Rückflußkühler mit den Reaktoren verbunden sind, je einem bis zur Mitte des Kolbens reichenden Zulaufrohr für frische Amino- und Carbonylverbindung, und gegebenenfalls Mutterlauge, je einem bis zur Mitte des Kolbens reichenden Zulaufrohr für frische und für gegebenenfalls rückgeführte Blausäure und je einem bis zum Boden reichenden Abflußrohr und einem Rohrreaktor, der mit 10 Lochböden versehen und am Ausgang mit einem Druckhalteventil abgeschlossen ist. Alternativ zu den Lochböden können in den Rohrreaktor auch Packungen, beispielsweise aus Sieb- oder Raschigringen, eingebaut werden.

Die Kolben sind mit thermostatisierten Wasserbädern versehen.

Ferner weist der Versuchsaufbau noch mit Rührern versehene Mischgefäße auf, in denen die NH-Funktionen aufweisende Verbindung und die Carbonylverbindung vor ihrer Einspeisung in die Kolben mit Wasser oder Mutterlauge vorgemischt werden können. Die Mischgefäße haben Zuläufe für frische Reaktanden und für rückgeführte Mutterlauge.

Zur Förderung des Reaktionsgemisches bzw. der frischen Reaktanden-Gemische dienen Labor-Dosierpumpen und zur Temperatureinstellung des Reaktionsgemisches vor dem Einspeisen in den Rohrreaktor ist ein gegebenenfalls zuschaltbarer Wärmetauscher zwischen dem zweiten Vierhalskolben und dem Rohrreaktor vorgesehen.

Der Ausgang des Rohrreaktors mündet in eine Aufarbeitungseinrichtung. Je nach dem aktuellen Produkt besteht die Aufarbeitungseinrichtung z.B. aus einem drucklos verschlossenen, kühlbaren, gerührten Kristallisationsgefäß, an das eine Drucknutsche angeschlossen ist, und/oder z.B. aus einem oder mehreren, gewünschtenfalls evakuierbaren Rotationsverdampfern mit nachgeschalteter Kühlfalle zur Kondensation überschüssiger Blausäure.

Die Aufarbeitungseinrichtung weist Auslässe für das hergestellte Produkt, für rückzuführende Mutterlauge und für rückzuführende Blausäure auf.

Auch kann Blausäure aus Mutterlauge durch nachfolgende Abdestillation, z.B. in einem der oben genannten Rotationsverdampfer, rückgewonnen und über dem Prozeß wieder zugeführt werden.

Ein Auslaß der Aufarbeitungsanlage kann je nach Bedarf mit den Einlässen der Reaktoren verbunden werden, ein Auslaß der Aufarbeitungsanlage kann je nach Bedarf mit den Einlässen der Reaktoren verbunden werden.

Über die Einlässe können auch bei Bedarf Zuschlagsstoffe zur pH-Regulierung, vorzugsweise anorganische Säuren, wie z.B. Schwefelsäure zugegeben werden. Die Zugabe erfolgt pH-geregelt im Zusammenhang mit den pH-Messungen durch die Elektroden.

**Beispiel 1**

Herstellung von Iminodiacetonitril

Der erste Vierhalskolben der Versuchsanlage wird mit einer Mischung von 1412 g 15,9 gew.-%iger wässriger Hexamethylentetramin (HMTA)-Lösung (~140 g HMTA), - hergestellt durch vorherige Umsetzung von 960 g 30 gew.-%igem wäßrigem Formaldehyd und 452 g 25 gew.-%igem wäßrigem Ammoniak und Nachreaktion für eine Stunde bei 50°C - und 277 g Blausäure (99 gew.-%ig) beschickt und durch Kühlung auf 50°C gehalten. Durch Zugabe von insgesamt 186 g 50 gew.-%iger wäßriger Schwefelsäure wird ein pH-Wert von 6 gehalten.

Man rührt 60 Minuten und beginnt dann, den Kolbeninhalt durch das Abflußrohr mit einer Geschwindigkeit von 1800 ml pro Stunde in den zweiten Kolben hinüber zu pumpen. Gleichzeitig wird dem ersten Kolben über das erste Zulaufrohr aus dem Mischer eine 15,9 gew-%ige Lösung von HMTA in Wasser oder Mutterlauge in einer Geschwindigkeit von 1410 ml pro Stunde und durch das zweite Zulaufrohr 277 g pro Stunde 99 %-ige Blausäure zudosiert.

Gleichzeitig beginnt man auch im zweiten Kolben durch Zugabe von 50 gew.-%iger Schwefelsäure den pH-Wert bei 6,0 zu halten.

Nachdem der Zulauf vom ersten in den zweiten Kolben eine Stunde lang erfolgt ist, ist im zweiten Kolben, der ebenfalls auf einer Temperatur von 50°C gehalten wird, ein Volumen von 1800 ml Reaktionsgemisch vorhanden.

Man beginnt nun, den Inhalt dieses zweiten Kolbens mit einer Geschwindigkeit von 1700 bis 1800 ml pro Stunde über einen Wärmetauscher, der bei 95°C betrieben wird, von unten in den Rohrreaktor (100 cm Länge und 1,8 cm lichte Weite) einzuspeisen.

Der Innendruck des Reaktors wird mittels eines Druckhalteventils auf 200 HPa Überdruck eingestellt. Sobald dieser Druck erreicht ist, öffnet sich das Ventil am Ausgang soweit, daß dieser Druck gehalten wird. Nach 60 Minuten tritt aus dem Druckhalteventil das Reaktionsgemisch aus, das wie folgt aufgearbeitet werden kann:

Je 2 1 des aus dem Reaktor austretenden Gemisches werden unter ständigem Rühren, z.B. in einem Rotationsverdampfer im Wasserstrahlvakuum bei 25°C bis zur beginnenden Kristallisation eingeengt und anschließend bei 5°C 30 Minuten ausgerührt. Der Niederschlag wird abgesaugt, das Filtrat (Mutterlauge) zur Herstellung des dem ersten Kolben zugeführten feeds eingesetzt. Der Niederschlag, bestehend aus Iminodiacetonitril, wird dann mit entsalztem Wasser gewaschen und getrocknet. Die in der Kühlfalle der Rotationsverdampfer aufgefangene Blausäure wird gewogen und über Zulauf in den Kolben wieder eingespeist.

Die Ausbeute beträgt, bezogen auf Blausäure, 97 % der Theorie.

**Beispiel 2**

Herstellung von Nitrilotriacetonitril.

Der erste Vierhalskolben der Versuchsanlage wird mit 1300 g einer 8,1 gew.-%igen wäßrigen Lösung von Hexamethylentetramin - hergestellt durch Mischen von 260 g 38,5 gew.-%iger Ammoniumsulfat-Lösung, 85 g 30 gew.-%igem wäßrigem Ammoniak und 955 g 30 gew.-%igem wäßrigen Formaldehyd bei 50°C und 1 Stunde Nachreaktion bei 50°C - und 260 g Blausäure (99 %ig) beschickt, durch Zusatz von 8 g 96 gew.-%iger Schwefelsäure auf einen pH-Wert von 0,5 eingestellt und auf eine Temperatur von 40 bis 50°C temperiert. Man rührt 45 Minuten und beginnt dann den Kolbeninhalt durch das Abflußrohr mit einer Geschwindigkeit von 32 ml pro Minute in den zweiten Kolben hinüber zu pumpen. Gleichzeitig wird dem ersten Kolben über das erste Zulaufrohr aus dem Mischer eine mit ca. 8 Gew.-Teilen 96 gew.-%iger Schwefelsäure auf pH 0,5 eingestellte Mischung von 1300 Gew.-Teilen einer 8,1 gew.-%igen wäßrigen Lösung von Hexamethylentetramin und 230 Gew.-Teilen Wasser oder Mutterlauge in einer Geschwindigkeit von 25 ml pro Minute und durch das zweite Zulaufrohr 4,3 g pro Minute 99 %-ige Blausäure zudosiert. In den zweiten Kolben wird gleichzeitig in einer Geschwindigkeit von 2 g pro Minute Wasser bzw. Mutterlauge oder Waschwasser aus der Produktabtrennung im Aufarbeitungsgefäß zudosiert.

Nach 45 Minuten ist in dem zweiten Kolben, der auf eine Temperatur von 50 bis 60°C eingestellt wird, ein Volumen von ca. 2000 ml Reaktionsgemisch vorhanden. Man beginnt nun, den Inhalt dieses zweiten Kolbens mit einer Geschwindigkeit von ca. 32 ml pro Minute von unten in den Rohrreaktor (50 cm Länge und 1,8 cm lichte Weite) einzuspeisen. Zwischen dem zweiten Kolben und dem Rohrreaktor ist hier ein Wärmetauscher geschaltet, in dem das hindurchfließende Reaktionsgemisch auf eine Temperatur von 110 bis 115°C erwärmt wird. Sobald der Innendruck des Reaktors 200 HPa erreicht, öffnet sich das Druckhalteventil am Ausgang soweit, daß dieser Druck gehalten wird. Nach 15 Minuten tritt aus dem Druckhalteventil das Reaktionsgemisch aus, das gesammelt, 30 Minuten bei 5 bis 10°C ausgerührt und auf einer Drucknutsche abfiltriert wird. Das Filtrat (Mutterlauge) wird teils zur Herstellung des dem ersten Kolben zugeführten feeds eingesetzt, teils zur Rückgewinnung überschüssiger Blausäure destilliert. Der Niederschlag, bestehend aus Nitrilotriacetonitril, wird dann mit entsalztem Wasser gewaschen und getrocknet. Die zurückgewonnene Blausäure wird gewogen und über Auslaß und Zulauf in den Kolben wieder eingespeist. Die Ausbeute beträgt, bezogen auf Blausäure, 98,3 % der Theorie.

**Beispiel 3**

Herstellung von Sarcosinnitril

Der erste Vierhalskolben der Versuchsanlage wird mit einer Lösung von 326 g Monomethylamin in 489 ml Wasser, 732 g einer 30 gew.%-igen wässrigen Formaldehydlösung und 202 g Blausäure (99 %ig) beschickt und durch Kühlung auf 30°C gehalten.Man rührt 2,5 Minuten und beginnt dann den Kolbeninhalt durch das Abflußrohr mit einer Geschwindigkeit von 60 ml pro Minute in den zweiten Kolben hinüber zu pumpen. Gleichzeitig wird dem ersten Kolben über das Zulaufrohr aus dem Mischer eine Mischung von 326 Gew.-Teilen Monomethylamin, 489 Gew.-Teilen Wasser und 732 Gew.-Teilen einer 30 gew.%-igen wässrigen Formaldehydlösung mit einer Geschwindigkeit von 53 ml pro Minute und durch das Zulaufrohr 6,7 g pro Minute 99 %-ige Blausäure zudosiert.

30 Minuten nach Beginn des Zulaufs vom ersten in den zweiten Kolben ist im zweiten Kolben, der ebenfalls auf einer Temperatur von 25°C gehalten wird, ein Volumen von ca. 1800 ml Reaktionsgemisch vorhanden. Man beginnt nun, den Inhalt dieses zweiten Kolbens mit einer Geschwindigkeit von 60 bis 65 ml pro Minute über den Auslauf und den Wärmetauscher, der bei 50°C betrieben wird, von unten in den Rohrreaktor (60 cm Länge und 1,8 cm lichte Weite) einzuspeisen. Sobald der Innendruck des Reaktors 200 HPa erreicht, öffnet sich das Druckhalteventil am Ausgang soweit, daß dieser Druck gehalten wird.

Nach 8 bis 10 Minuten tritt aus dem Druckhalteventil das Reaktionsgemisch aus, das ohne weitere Aufreinigung in einer Folgestufe direkt zum Sarkosin weiterverarbeitet werden kann. Die Ausbeute beträgt, bezogen auf Blausäure, 95,7 % der Theorie.

**Beispiel 4**

Herstellung von N,N-Biscyanmethyl-2-aminopropionitril

Der erste Vierhalskolben der Versuchsanlage wird mit 1132 g einer 50°C warmen Mischung von 26 Gew.-%

Iminodiacetonitril in Mutterlauge, wie sie in Beispiel 1 am Ausgang des Rohrreaktors anfällt (oder alternativ dazu von 294 g Iminodiacetonitril in 838 g Wasser), 163 g Acetaldehyd und 92,8 g Blausäure (99 %ig) beschickt, durch Zusatz von ca. 98 g 50 gew.-%iger Schwefelsäure auf einen pH-Wert von 1,5 eingestellt und auf eine Temperatur von 50°C temperiert. Man rührt 20 Minuten und beginnt dann den Kolbeninhalt durch das Abflußrohr mit einer Geschwindigkeit von 75 ml pro Minute in den zweiten Kolben hinüber zu pumpen. Gleichzeitig wird dem ersten Kolben aus dem Mischer über das Zulaufrohr eine mit Schwefelsäure auf pH 1,5 eingestellte Mischung von 838 Gew.-teilen Wasser oder Mutterlauge, 294 Gew.-teilen Iminodiacetonitril, 98 Gew.-teilen 50 gew.-%iger Schwefelsäure und 163 Gew.-teilen Acetaldehyd in einer Geschwindigkeit von 70 ml pro Minute und durch das zweite Zulaufrohr 4,7 g pro Minute 99 %-ige Blausäure zudosiert.

Nach 20 Minuten ist in dem zweiten Kolben, der auf eine Temperatur von 50 bis 60°C eingestellt wird, ein Volumen von ca. 1500 ml Reaktionsgemisch vorhanden. Man beginnt nun, den Inhalt dieses zweiten Kolbens mit einer Geschwindigkeit von ca. 75 ml pro Minute von unten in den Rohrreaktor (50 cm Länge und 1,8 cm lichte Weite) einzuspeisen. Zwischen dem zweiten Kolben und dem Rohrreaktor ist hier ein Wärmetauscher geschaltet, in dem das hindurchfließende Reaktionsgemisch auf eine Temperatur von 70°C erwärmt wird. Sobald der Innendruck des Reaktors 200 HPa erreicht, öffnet sich das Druckhalteventil am Ausgang soweit, daß dieser Druck gehalten wird. Nach 20 Minuten tritt aus dem Druckhalteventil das Reaktionsgemisch aus, das gesammelt, 90 Minuten bei 40°C und 30 Minuten bei 5 bis 10°C ausgerührt und auf einer Drucknutsche abfiltriert wird. Das Filtrat (Mutterlauge) wird teils zur Herstellung des dem ersten Kolben zugeführten feeds eingesetzt, teils zur Rückgewinnung überschüssiger Blausäure destilliert.

Der Niederschlag, bestehend aus N,N-Biscyanmethyl-2-aminopropionitril, wird dann mit entsalztem Wasser gewaschen und getrocknet. Die zurückgewonnene Blausäure wird gewogen und in den Kolben wieder eingespeist. Die Ausbeute beträgt, bezogen auf Blausäure, 98,5 % der Theorie.

**Patentansprüche**

1. Verfahren zur Cyanoalkylierung von Ammoniak oder organischen Verbindungen mit einer oder mehreren NH-Funktionen durch Umsetzung besagter Verbindungen mit einer Carbonylverbindung und Blausäure in einem geeigneten Lösungsmittel, **dadurch gekennzeichnet, daß** eine NH-Funktionen enthaltende Verbindung der Formel I

$$R_a-NH_{(3-a)} \tag{I},$$

worin R ein gegebenenfalls substituierter linearer, verzweigter oder cyclischer Alkylrest mit 1 bis 8 C-Atomen oder Aryl mit 6 bis 10 C-Atomen ist, a eine der Ziffern 0, 1 oder 2 bedeutet und die Verbindung der Formel I insgesamt 1 bis 8 Kohlenstoffatome hat, mit einem Aldehyd der Formel II

$$R^2-CHO \tag{II},$$

worin $R^2$ Wasserstoff oder ein gegebenenfalls substituierter linearer oder verzweigter Alkylrest mit 1 bis 6 C-Atomen ist im Molverhältnis Verbindung der Formel I zu Verbindung der Formel II von 0,2 bis 2,0 umsetzt, wobei die Reaktion zunächst in einem oder mehreren kaskadierten Reaktoren drucklos bei einer Temperatur $T_1$ von unter 85°C bis zu einem Umsetzungsgrad von mindestens 60 %, bezogen auf Blausäure, und anschließend in einem oder mehreren kaskadierten unter Druck arbeitenden Reaktoren bei einer Temperatur $T_2$, die unterhalb 150°C liegt, bis zu einem Umsetzungsgrad von mindestens 90 %, bezogen auf Blausäure, ausgeführt wird.

2. Verfahren gemäß Anspruch 1, **dadurch gekennzeichnet, dass** R in Formel I lineares Alkyl mit 1 bis 4 C-Atomen oder Phenyl bedeutet und der Index a, sofern R Alkyl ist, für 1 oder 2, sofern R ein Arylrest ist, für die Ziffer 1 steht.

3. Verfahren gemäß den Ansprüchen 1 und 2, **dadurch gekennzeichnet, dass** als Carbonylverbindung Formaldehyd, Acetaldehyd oder Propionaldehyd eingesetzt wird.

4. Verfahren gemäß den Ansprüchen 1 bis 3, **dadurch gekennzeichnet, dass** im ersten Reaktionsabschnitt die Umsetzung bis zu einem Umsetzungsgrad von 60 bis 85 %, im zweiten Reaktionsabschnitt bis zu einem Umsetzungsgrad von 90 bis 100 % getrieben wird.

5. Verfahren gemäß den Ansprüchen 1 bis 4, **dadurch gekennzeichnet, dass** für den zweiten Reaktionsabschnitt Rohrreaktoren eingesetzt werden, die zweckmäßigerweise mit Einbauten zur Verminderung der Rückmischung versehen sind, dass sie neben der Verhinderung der Rückmischung auch die Umsetzung inhomogener Reaktionsgemische erlauben.

6. Verfahren gemäß den Ansprüchen 1 bis 5, **dadurch gekennzeichnet, dass** das Molverhältnis von Carbonylverbindung zu Blausäure im Reaktionssystem im Bereich von 0,5 bis 1,2 gehalten wird.

**Claims**

1. A process for the cyanoalkylation of ammonia or organic compounds having one or more NH functions by reaction of said compounds with a carbonyl compound and hydrocyanic acid in a suitable solvent, which comprises reacting a compound of the formula I containing NH functions

$$R_a\text{-}NH_{(3\text{-}a)} \qquad (I)$$

where R is a substituted or unsubstituted linear, branched or cyclic alkyl radical having 1 to 8 carbon atoms or aryl having 6 to 10 carbon atoms, a is one of the numbers 0, 1 and 2, and the compound of the formula I has a total of from 1 to 8 carbon atoms with an aldehyde of the formula II

$$R^2\text{-}CHO \qquad (II)$$

where $R^2$ is hydrogen or a substituted or unsubstituted linear or branched alkyl radical having 1 to 6 carbon atoms, in a molar ratio of compound of formula I to compound of formula II of from 0.2 to 2.0, the reaction being carried out first in one or more cascaded reactors, unpressurized, at a temperature $T_1$ of below 85°C to a degree of conversion of at least 60%, based on hydrocyanic acid, and then in one or more cascaded, pressurized reactors at a temperature $T_2$ of below 150°C to a degree of conversion of at least 90%, based on hydrocyanic acid.

2. A process as claimed in claim 1, wherein R in the formula I is linear alkyl having 1 to 4 carbon atoms or is phenyl, and the index a is 1 or 2 if R is alkyl or the number 1 if R is an aryl radical.

3. A process as claimed in one of claims 1 and 2, wherein the carbonyl compound employed is formaldehyde, acetaldehyde or propionaldehyde.

4. A process as claimed in one of claims 1 to 3, wherein the reaction in the first reaction section is carried out to a degree of conversion of from 60 to 85%, and the reaction in the second reaction section is carried out to a degree of conversion of from 90 to 100%.

5. A process as claimed in one of claims 1 to 4, wherein the second reaction section is carried out using tubular reactors which are advantageously provided with internals for reducing back-mixing, such that, in addition to preventing back-mixing, they also allow the reaction of inhomogeneous reaction mixtures.

6. A process as claimed in one of claims 1 to 5, wherein the molar ratio between carbonyl compound and hydrocyanic acid in the reaction system is held in the range from 0.5 to 1.2.

**Revendications**

1. Procédé de cyanoalkylation d'ammoniac ou de composés organiques comportant une ou plusieurs fonctions OH par réaction desdits composés avec un composé carbonyle et de l'acide cyanhydrique dans un solvant approprié, **caractérisé en ce que** l'on fait réagir un composé de formule I contenant une fonction NH

$$R_3\text{-}NH_{(3\text{-}a)} \qquad (I)$$

dans laquelle R est un radical alkyle linéaire, ramifié ou cyclique éventuellement substitué comportant de 1 à 8 atomes de C ou aryle comportant de 6 à 10 atomes de C, a représente le chiffre 0, 1 ou 2 et le composé de formule I possède au total de 1 à 8 atomes de carbone, avec un aldéhyde de formule II

$$R^2\text{-CHO} \tag{II}$$

dans laquelle $R^2$ représente de l'hydrogène ou un radical alkyle linéaire ou ramifié éventuellement substitué comportant de 1 à 6 atomes de C, en proportion molaire du composé de formule I au composé de formule II de 0,2 à 2,0, la réaction étant entreprise d'abord dans un ou plusieurs réacteurs en cascade, hors pression, à une température $T_1$ de moins de 85°C jusqu'à un taux de conversion d'au moins 60%, par rapport à l'acide cyanhydrique, et ensuite dans un ou plusieurs réacteurs en cascade travaillant sous pression, à une température $T_2$ située au-dessous de 150°C, jusqu'à un taux de conversion d'au moins 90%, par rapport à l'acide cyanhydrique.

2.  Procédé selon la revendication 1, **caractérisé en ce que** dans la formule I, R représente un radical alkyle linéaire comportant de 1 à 4 atomes de C ou phényle, et l'indice a, dans la mesure ou R est un alkyle, a une valeur de 1 ou 2, et représente le chiffre 1 lorsque R est un radical aryle.

3.  Procédé selon les revendications 1 ou 2, **caractérisé en ce que** l'on met en oeuvre en tant que composé carbonyle du formaldéhyde, de l'acétaldéhyde ou de l'aldéhyde propionique.

4.  Procédé selon les revendications 1 à 3, **caractérisé en ce que**, dans la première étape de réaction, on conduit la réaction jusqu'à un taux de conversion de 60 à 85%, et dans la deuxième étape de réaction jusqu'à un taux de conversion de 90 à 100%.

5.  Procédé selon les revendications 1 à 4, **caractérisé en ce que** l'on met en oeuvre, pour la deuxième étape de réaction, des réacteurs tubulaires, qui sont avantageusement munis d'éléments encastrés destinés à réduire le remélange, et tels qu'outre une réduction du remélange, ils permettent aussi la réaction de mélanges réactionnels inhomogènes.

6.  Procédé selon les revendications 1 à 5, **caractérisé en ce que** la proportion molaire du composé carbonyle à l'acide cyanhydrique dans le système réactionnel est maintenue dans la plage de 0,5 à 1,2.